(19) **Europäisches Patentamt**
**European Patent Office**
**Office européen des brevets**

(11) **EP 3 467 972 B1**

(12) **EUROPEAN PATENT SPECIFICATION**

(45) Date of publication and mention
of the grant of the patent:
**19.04.2023 Bulletin 2023/16**

(21) Application number: **18198565.6**

(22) Date of filing: **04.10.2018**

(51) International Patent Classification (IPC):
**H01S 3/0941** *(2006.01)*  **H01S 3/00** *(2006.01)*
**G02B 6/38** *(2006.01)*  **G02B 6/42** *(2006.01)*
*A61B 18/22* *(2006.01)*  *H01S 3/094* *(2006.01)*
*H01S 3/16* *(2006.01)*  *A61B 18/00* *(2006.01)*
*A61N 5/06* *(2006.01)*  *A61B 90/00* *(2016.01)*

(52) Cooperative Patent Classification (CPC):
**H01S 3/005; G02B 6/4296; H01S 3/0941;**
A61B 18/22; A61B 90/70; A61B 2018/0097;
A61B 2018/00988; A61B 2090/0813;
A61N 2005/063; H01S 3/094076; H01S 3/161;
H01S 3/1643

(54) **METHOD FOR OPERATING A PULSED LASER SYSTEM**

VERFAHREN ZUM BETRIEB EINES GEPULSTEN LASERSYSTEMS

PROCÉDÉ DE FONCTIONNEMENT D'UN SYSTÈME LASER PULSÉ

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **04.10.2017 DE 102017217572**

(43) Date of publication of application:
**10.04.2019 Bulletin 2019/15**

(73) Proprietor: **Dornier MedTech Laser GmbH
82234 Wessling (DE)**

(72) Inventors:
• **Hiereth, Werner
92262 Birgland (DE)**

• **Rampp, Michael
81375 München (DE)**
• **Sperl, Alexander
13156 Berlin (DE)**

(74) Representative: **Grünecker Patent- und
Rechtsanwälte
PartG mbB
Leopoldstraße 4
80802 München (DE)**

(56) References cited:
**US-A- 5 269 778**    **US-A1- 2006 089 629**
**US-A1- 2008 262 577**    **US-A1- 2009 130 622**

## Description

### TECHNICAL FIELD

The invention relates to a method for operating a pulsed laser system according to the preamble of claim 1 and to a pulsed laser system according to the preamble of claim 10.

### BACKGROUND AND SUMMARY

Usually, a laser resonator of a pulsed laser system is pumped by means of a pump source in order to generate a laser beam of operating laser pulses at a certain operating energy level. In order to apply the operating laser pulses to a target area, such as inside a human body, the laser beam needs to be coupled into an optical fiber by means of a focusing element and guided via the optical fiber to the target area. For example the focusing element can comprise a coupling lens or a coupling mirror.

However, in order to efficiently couple the laser beam into the optical fiber, a focusing element with high optical quality and a short focal length is needed. Thereby the laser beam is focused on to a small focal point, where a proximal end face of the optical fiber is situated. Consequently, the proximal end face of the optical fiber is in close proximity to the focusing element.

In case the optical fiber is contaminated, e.g. when a reusable optical fiber is not cleaned, disinfected and/or sterilized according to the operating manual, the contaminations on the fiber can absorb the laser beam and get accelerated towards the focusing element whereby it is being sputtered. This dramatically worsens the optical quality and the focusing ability of the focusing element. Thus, the waist of the laser beam increases at the proximal end face of the optical fiber. As soon as the focused laser beam reaches the cladding of the optical fiber, a large amount of energy is dissipated into structures surrounding the proximal end of the optical fiber, such as a buffer or a ferrule. As a consequence, hot glue particles of said structures are sputtered onto the focusing element as well. The described effect leads to a vicious cycle which damages the focusing element so much that it needs to be replaced by a new focusing element which then needs realignment with the laser resonator and the optical fiber.

Document US 9,122,009 B1 discloses fiber optic terminations for discriminating between potentially damaging energy and energy that has potential utility, wherein the potentially damaging energy is dissipated within the fiber optic termination. However, even though the proposed fiber optic terminations increase the lifetime of the optical fiber, they do not prevent the sputtering of the coupling lens when the optical fiber is contaminated.

Document CN 101867142 A discloses a protective lens for a laser coupling mirror of a medical holmium laser treatment machine, which comprises a lens body placed between the coupling mirror and the optical fiber. However, even though this prevents the coupling lens from being sputtered, still the protective lens degrades by being sputtered, which then needs replacement. Moreover, this approach does not prevent the optical fiber from being degraded by the distorted laser beam.

Therefore, it is an object of the invention to provide a method and a pulsed laser system which is improved with respect to the degradation of the focusing element and the optical fiber caused by contaminations.

Document US 2008/0262577 A1 discloses a method and apparatus for treatment of solid material including hard tissue.

Document US 5,269,778 discloses a variable pulse width laser and method for use.

To solve this problem the invention provides a method for operating a pulsed laser system with the features of claim 1. Advantages embodiments are mentioned in this sub claims.

According to the invention, the optical fiber is cleaned prior to generating the operating laser pulses by means of the cleaning laser pulses, wherein the control unit controls the pump source at one or more cleaning voltages and/or one or more cleaning currents such that pump energy is provided to the laser resonator at one or more low levels, wherein the laser resonator of the pulsed laser system is pumped by means of the pump source in order to generate the cleaning laser pulses at one or more cleaning energy levels between the laser threshold and the operating energy level, wherein the pump pulse duration for the cleaning laser pulses is at least 600 $\mu$s, and wherein at least part of a plurality of contaminations is removed from the proximal end face of the optical fiber. Thus, the cleaning laser pulses contain less energy than the operating laser pulses and, therefore, less energy is dissipated as heat in the contaminations or the cladding at the proximal end face of the optical fiber. Consequently, particles still get accelerated away from the proximal end face, however, with less kinetic energy. Thus, the proximal end face of the optical fiber is cleaned by the cleaning laser pulses without sputtering the focusing element.

The pulsed laser system can be a medical pulsed laser system, for example a surgical laser system. Particularly, the pulsed laser system can be a holmium laser system (Ho:YAG-laser). The pump source can comprise at least one light source such as a pump laser system or a conventional light source. For example, the pump laser system can be a semi conductor laser or the conventional light source can be a flashbulb or an LED. In at least one example the pump source can be configured to generate pump pulses. The pump source can be coupled to the laser resonator by means of a pump energy coupling element, for example a coupling mirror.

The laser resonator can comprise at least two reflecting surfaces and a laser rod, for example a holmium crystal. One of the reflecting surfaces can be configured to be fully-reflecting and the other one of the reflecting surfaces can be configured to be semi-reflecting. The semi-reflecting surface can be an exit surface for the operating laser pulses towards to the focusing element. In at least one example the reflecting surfaces can be embodied as mirrors. In at least one example, the laser resonator can comprise a master resonator with the laser rod and with the at least two reflecting surfaces and an amplification stage with a further laser rod coupled to the master resonator via one of the at least two reflecting surfaces. In this case laser pulses from the master resonator may be amplified by the amplification stage to generate the operating laser pulses and/or the cleaning laser pulses. The pump source may comprise at least one first light source for the master resonator and at least one second light source for the amplification stage. The laser resonator may comprise a Q-switch for controlling the pulse duration of at least one of the operating laser pulses and/or the cleaning laser pulses.

The focusing element can comprise a coupling lens or a coupling mirror. It is also conceivable that the focusing element comprises several optical elements in order to focus the operating laser pulses onto the optical fiber.

The optical fiber can comprise or be any type of waveguide for laser light, such as a glass fiber or a plastic fiber. The optical fiber can be a monomode fiber or a multimode fiber. The optical fiber comprises a proximal end face for coupling the laser beam of the operating laser pulses and the cleaning laser pulses into the optical fiber. The proximal end face of the optical fiber can be directed to the focusing element and/or a distal end face of the optical fiber can be directed to a target area. Moreover, the optical fiber may comprise a connector element at the proximal end face in order to releasably attach it to a mounting bracket. Thereby, the optical fiber may be easily replaced or removed from the pulsed laser system. The optical fiber can be embodied as a single-use or as a reusable fiber. The distal end face of the optical fiber may be embedded into an operating head which may be used by an operator to irradiate the laser light on to the target area.

"Pumping the laser resonator" can mean in this context to provide energy to the laser resonator causing the laser rod to emit laser light via spontaneous or stimulated emission. In at least one example the laser resonator is provided with light energy during pumping.

"Generating operating laser pulses" can mean to generate a sequence of laser pulses at the operating energy level which is suitable to treat the target area. The operating energy level is in a range from 100 mJ to 3000 mJ or in at least one example in a range from 200 mJ to 2000 mJ.

"Cleaning the optical fiber" can mean in this context to remove at least part of a plurality of contaminations from the proximal end face of the optical fiber.

"Laser threshold" can mean that the laser resonator starts emitting laser energy at the lowest possible energy level when it is pumped by the pump source at the laser threshold.

The operating energy level and the clean energy level may refer to a peak energy level of the respective laser pulses.

The one or more cleaning energy levels can be in a range from the laser threshold to below the operating energy level. In at least one example the one or more cleaning energy levels can be in a range from the laser threshold to 10mJ below the operating energy level.

The pump pulse duration for the cleaning pulses is at least $600\mu s$, in at least one example at least $1000\mu s$. A longer pulse duration of the at least one cleaning laser pulses leads to a lower peak power, whereby the contaminations are accelerated less than with higher peak power.

If the operating energy level is less than a maximum cleaning energy level, then the one or more cleaning energy levels can be in a range from the laser threshold to the operating energy level and if the operating energy level is equal to or above the maximum cleaning energy level, then the one or more cleaning energy levels can be in a range from the laser threshold to the maximum cleaning energy level, wherein the maximum cleaning energy level is in a range from 400 to 1000 mJ. Consequently, if the user sets the operating energy level equal to or above the maximum cleaning energy level, then the cleaning laser pulses are limited to the maximum cleaning energy level for protecting the optical fiber against high energy particles and, furthermore, if the user sets the operating energy level below the maximum cleaning energy level, then the energy of the cleaning laser pulses does not unnecessarily exceed the operating energy level actually used. In at least one example the maximum cleaning energy level can be in a range from 500 mJ to 600 mJ. The maximum cleaning energy level may be an energy level, where the focusing element is not contaminated by accelerated particles from the optical fiber.

If the operating energy level is below the maximum cleaning energy level, the method may comprise a second step of cleaning the optical fiber after generating the first operating laser pulses by means of second cleaning laser pulses, wherein the laser resonator of the pulsed laser system is pumped by means of the pump source in order to generate the second cleaning laser pulses at one or more second cleaning energy levels between the first operating energy level and a second operating energy level, wherein the second operating energy level is higher than the first operating energy level. The method may comprise a step of pumping the laser resonator of the pulsed laser system by means of the pump source after the second step of cleaning the optical fiber in order to generate second operating laser pulses at the second operating energy level. Thus, the optical fiber is not damaged during a second usage at the higher second operating energy level.

The one or more cleaning energy levels can be between the laser threshold and one half or one third of the operating energy level. Thus, the cleaning level is even lower and the risk of the focusing element being sputtered on is further reduced.

The cleaning laser pulses may be generated as a sequence of laser pulse groups, wherein each one of the laser pulse groups comprises at least one of the cleaning laser pulses, and wherein the cleaning energy level is increased from one laser pulse group to a following laser pulse group. By systematically increasing the cleaning energy level from one laser pulse group to the following laser pulse group, the energy to remove and/or vaporize the contaminations is ramped up very smoothly. The lower the energy which is used for different mass of particles, the less energy is deposited in the contamination and the less is the acceleration of the removed/vaporized contamination. Thus, even if a laser pulse group with a high cleaning energy level is coupled into the optical fiber, the contaminations are already removed by a prior laser pulse group with a lower cleaning energy level. Consequently, the risk of the focusing element being sputtered is even further reduced. Each one of the laser pulse groups may comprise only one cleaning laser pulse or a plurality of cleaning laser pulses. If each one of the laser pulse groups comprises a plurality of cleaning laser pulses, the cleaning energy is further distributed in time in order to reduce the risk of the focusing element being sputtered by high-energy particles.

The cleaning energy level may be increased by a linear factor and/or an exponential factor from one laser group to the following laser group. As an example, a pump voltage for driving the pump source may be increased for at least part of the laser pulse groups from one laser pulse group to a following laser pulse group by a constant delta voltage. The constant delta voltage may be in the range of 1V - 10V or in at least one example in the range of 2V - 5V. As another example, the pump voltage may be increased according to $e^{(n/C)}$, wherein n is a number of the laser pulse group and C is a constant value. The constant value C may be in the range from 1 to 10 or in at least one example in the range from 3-4.

Each one of the laser pulse groups may comprise at least two of the cleaning laser pulses at the same cleaning energy level. In at least one example each one of the laser pulse groups may include only cleaning laser pulses of the same cleaning energy level. Consequently, the cleaning energy level is increased from one laser pulse group to the following laser pulse group in a step-like manner.

When the optical fiber is a reusable optical fiber, a data storage device associated with it may be readout in order to detect a prior usage of the reusable optical fiber, and wherein the step of cleaning the optical fiber is only carried out, if the prior usage of the reusable optical fiber is detected. Consequently, the reusable optical fiber is not cleaned directly after the production process where it is cleaned anyway. The data storage device may be associated with the connector elements at the proximal end face of the optical fiber. The data storage device may comprise an electronic memory. The method may comprise a step of storing a usage-information-tag into the data storage device after coupling the operating laser pulses with the focusing element into the optical fiber.

The method may further comprise prior to cleaning the optical fiber a step of heating up the laser rod of the pulsed laser system by means of pre-pumping-pulses of the pump source, wherein the pump source is operated below the laser threshold. Thereby, the laser rod is heated up prior to the cleaning step so that it operates at a stable condition.

The pump source may be controlled by the control unit selectively at the operating voltage and/or operating current or at the one or more cleaning voltages and/or one or more cleaning currents such that the pump energy is provided to the laser resonator selectively at a the high level for generating the operating laser pulses at the operating energy level or at the one or more low levels for generating the cleaning laser pulses at the one or more cleaning energy levels. In other words the control unit may selectively drive the pump source such that pump energy is provided to the laser resonator selectively at a high level for generating the operating laser pulses at the operating energy level or at one or more low levels for generating the cleaning laser pulses at the one or more cleaning energy levels. Thus, the sequence of the laser pulse groups can be easily implemented by a control function in the control unit. The control unit can be configured to readout the data storage device associated with the reusable optical fiber.

In addition, the invention provides a pulsed laser system with the features of claim 10. A further embodiment is mentioned in the sub-claim.

According to the invention, the control unit is configured to control the pump source at one or more cleaning voltages and/or one or more cleaning currents such that the pump energy is provided to the laser resonator at one or more low levels for generating cleaning laser pulses prior to the operating laser pulses at one or more cleaning energy levels between a laser threshold and the operating energy level for cleaning the optical fiber in order to at least partly remove a plurality of contaminations from the proximal end face of the optical fiber, wherein the pump pulse duration for the cleaning laser pulses is at least 600 μs. Thus, the optical fiber is cleaned prior to generating the operating laser pulses by means of the cleaning laser pulses. Since the cleaning laser pulses are generated at one or more cleaning energy levels between a laser threshold and the operating energy level, they contain less energy than the operating laser pulses. Thus, less energy is dissipated as heat in the contaminations or in the cladding of the proximal end face of the optical fiber. Consequently, particles still get accelerated from the proximal end face of the optical fiber, however, with less kinetic energy. Thus, the proximal end face of the optical fiber is cleaned by the cleaning laser pulses without sputtering the focusing element.

The pulsed laser system can be configured to perform the method for operating a pulsed laser system according to any one of claims 1 - 9.

## BRIEF DESCRIPTION OF THE FIGURES

Further features and advantages of the invention will be explained in greater detail in the following and based on the embodiment shown in the figures. The figures show:

Figure 1     a schematic overview of an embodiment of a pulsed laser system;
Figure 2     a flow chart of an embodiment of a method for operating the pulsed laser system in Figure 1; and
Figure 3     a diagram showing the energy of the cleaning laser pulses and the operating laser pulses.

## DETAILED DESCRIPTION

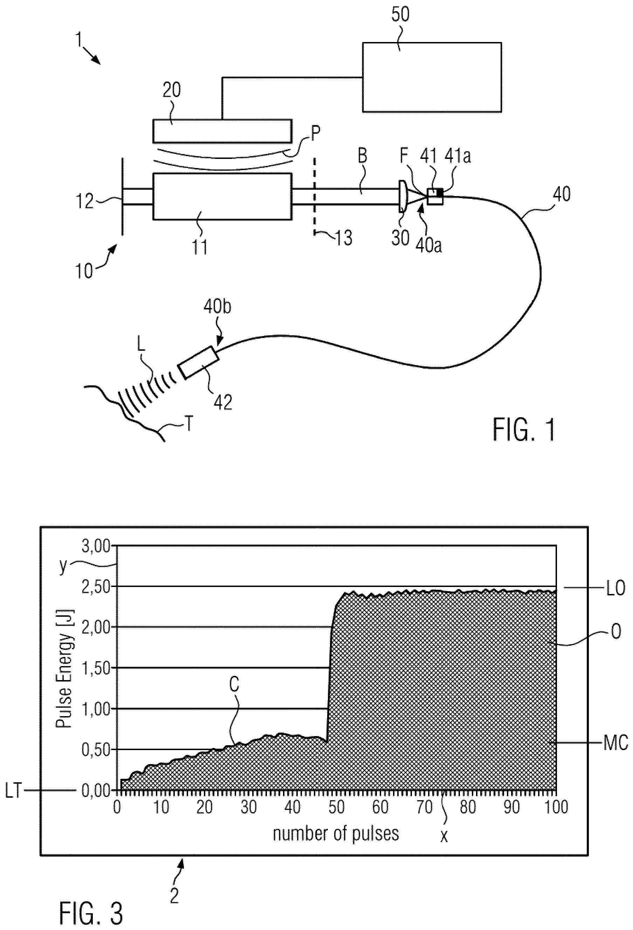

Figure 1 shows a schematic overview of an embodiment of a pulsed laser system 1. It shows the laser resonator 10 comprising the laser rod 11, the first mirror 12 having a fully reflecting surface and the second mirror 13 having a semi-reflecting surface. The laser rod 11 is for example a holmium crystal (Ho:YAG) which emits infrared light at a wavelength in the range of 2050 - 2150 nm when resonating. The pump energy P is supplied to the laser rod 11 in a pulsed manner by means of the pump source 20 which may be a flashbulb or a semi conductor laser arrangement. The pump source 20 is controlled by the control unit 50 selectively at an operating voltage or at several cleaning voltages such that pump energy P is provided to the laser rod 11 either at a high level for generating operating laser pulses at an operating energy level or at several low levels for generating cleaning laser pulses at several respective cleaning energy levels. Both the operating voltage and the cleaning voltages are controlled by the control unit 50 to form a pulsed signal at a repetition rate of, for example 20Hz. Thus, the operating laser pulses and the cleaning laser pulses have said repetition rate. Moreover, the laser resonator 10 may further comprise an amplification stage and a Q-switch.

The operating laser pulses and the cleaning laser pulses are emitted by the laser resonator 10 as the laser beam B which is focused by the focusing element 30 to form the focal point F. In this case the focusing element 30 is a coupling lens. However, it is conceivable that the focusing element 30 may be a coupling mirror or a combination of several optical elements.

Moreover, it can be further seen in Figure 1 that the proximal end face 40a of the optical fiber 40 is located at the focal point F in order to couple the laser beam B with the operating laser pulses and the cleaning laser pulses into the optical fiber 40. The proximal end face 40a is surrounded by a cladding and embedded in the connector element 41 which is releasably attached to a mounting bracket (not shown). In this case the optical fiber 40 is a reusable fiber which can be used by an operator several times. In order to detect the number of usages, the connector element 41 comprises a data storage device 41a which can be written to and read out by the control unit 50.

The distal end face 40b of the optical fiber 40 is embedded into an operating head 42 which may be used by the operator to irradiate the laser light L on to a target area T.

Since the beam waste needs to be very small at the focal point F in order to provide a high coupling efficiency, the focal distance of the focusing element 30 is as small as possible. Consequently, the proximal end face 40a of the optical fiber 40 is located close to the focusing element 30. Thus, there is a possible risk of contaminations at the proximal end face 40a being heated up by the operating laser pulses and being accelerated towards the focusing element 30. Consequently, the focusing element 30 may become sputtered, if the optical fiber 40 has not been cleaned appropriately.

In order to prevent sputtering of the focusing element 30 the proximal end face 40a of the optical fiber 40 is cleaned by means of cleaning pulses formed according to the method 100 described subsequently.

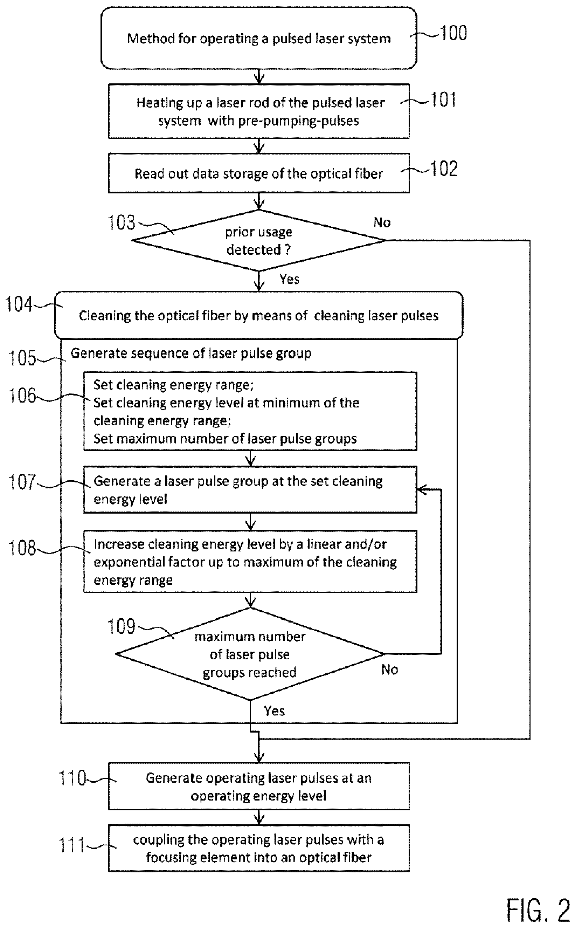

Figure 2 shows a flow chart of an embodiment of the method 100 for operating the pulsed laser system 1 in Figure 1.

As a first step 101 the laser rod 11 of the pulsed laser system 1 is heated up with a series of pre-pumping-pulses for stable operation. Therefore, the control unit 50 controls the pump source 20 with a series of pulses at a pre-pulse-voltage below a threshold voltage $U_{thr}$ which corresponds to the laser threshold. Therefore, the laser rod 11 is heated up without actually generating laser pulses.

As the next step 102 the data storage device 41a of the optical fiber 40 is read out by the control unit 50. If by the decision 103 a prior usage of the optical fiber 40 is not detected, it is new and has been sterilized appropriately after production. Therefore, cleaning is not necessary and the method further proceeds with step 110 as described below. However, if a prior usage of the optical fiber 40 is detected, the cleaning procedure 104 is performed by means of cleaning laser pulses.

In order to minimize the risk of contaminations being sputtered onto the focusing element 30, a sequence of laser pulse groups is generated according to the procedure 105 which is described in conjunction with Figure 3, which shows as diagram 2 the pulse number on the X-axis and the pulse energy in joules on the Y-axis for the cleaning laser pulses C and the subsequent operating laser pulses P.

As an initial step 106, a cleaning energy range is set. If the operating energy level LO is less than 600 mJ, then the cleaning energy range is set from just below the laser threshold LT to the operating energy level LO. However, if the operating energy level LO is equal to or above 600 mJ, then the cleaning energy range is set from just below the laser threshold LT to 600 mJ. Therefore, the maximum cleaning energy level MC possible is 600 mJ. Moreover, the cleaning laser energy level for the first cleaning laser pulses is set to the minimum of the cleaning energy range. As an example only, the maximum number of laser pulse groups is set to 25, wherein each pulse group has a number of two pulses.

Furthermore, the steps 107 - 109 are repeated until the maximum number of laser pulse groups is reached: In step 107 a first group of two cleaning laser pulses is generated at a cleaning energy level just below the laser threshold LT. As a next step 108 the cleaning energy level is subsequently increased for the first ten laser pulse groups by a first linear factor and an exponential factor and for the second ten laser pulse groups by a second linear factor, only. The cleaning energy level is increased up until the maximum of the cleaning energy range has been reached, in this case after the second ten laser pulse groups. For the last five laser pulse groups the cleaning energy level is held constant at the maximum of the cleaning energy range. In order to increase the cleaning energy levels, the cleaning voltages $U_{FL}$ applied to the pump source 20 are generated according to formulas (1), (2), wherein n is the index of the laser pulse group and $U_{thr}$ is the threshold voltage corresponding to the laser threshold:

$$n \in [1,10]: U_{FL}(n) = (U_{thr} - 5V) + 2.5V * n + e^{n/3.3} \qquad (1)$$

$$n \in [11,20]: U_{FL}(n) = U_{FL}(n-1) + 4V \qquad (2)$$

In other words the cleaning voltage $U_{FL}$ is increased for the first ten laser pulse groups by the first linear factor 2.5V and the exponential factor $e^{n/3.3}$ and for the second ten laser pulse groups by the second linear factor 4V.

It is understood, that the cleaning voltages $U_{FL}$ represents a peak voltage of a pulsed signal applied to the pump source 20. Within each laser pulse group, the peak voltage of the pulsed signal is constant and, therefore, the cleaning energy level within each laser pulse group is constant for each one of the cleaning laser pulses. For example, the pulsed signal may have a repetition rate of 20 Hz and the pump pulse duration is set to 700µs.

If the maximum number of laser pulse groups has been reached in step 109, the cleaning procedure is terminated and the operator may begin to generate the operating laser pulses at an operating energy level in step 110. As described above, the operating laser pulses are coupled with the focusing element 30 into the optical fiber 40 in step 110.

As a result, it can be seen in Figure 3 that during the cleaning procedure 104 - 109 the laser resonator 10 of the pulsed laser system 1 is pumped by means of the pump source 20 in order to generate the cleaning laser pulses C with increasing cleaning energy levels between the laser threshold LT and the maximum cleaning energy level MC of 600 mJ, wherein the cleaning laser pulses C are generated as a sequence of laser pulse groups. Furthermore, the operating laser pulses O are generated at the operating energy level LO.

Consequently, the optical fiber 40 is cleaned prior to generating the operating laser pulses O by means of cleaning laser pulses C, wherein the laser resonator 10 of the pulsed laser system 1 is pumped by means of the pump source 20 in order to generate the cleaning laser pulses C at cleaning energy levels between the laser threshold LT and the maximum cleaning energy level MC of 600 mJ. Since the cleaning laser pulses C have less energy than the operating laser pulses O, less energy is dissipated as heat in contaminations and the cladding at the proximal end face 40a of the optical fiber 40. Consequently, particles still get removed there from, however, with less kinetic energy. Thus, the proximal end face 40a of the optical fiber 40 is cleaned by the cleaning laser pulses C without sputtering the focusing element 30.

In a further example not shown in Figs. 1 - 3, the operating energy level is at 300 mJ below the maximum cleaning energy level of 600 mJ and a second operating energy level at 1000 mJ. Thus, if the optical fiber is only cleaned in steps 106 - 109 until the operating energy level of 300 mJ, it may become damaged when using it with the second operating energy level of 1000 mJ. Therefore, the method 100 may comprise a second step of cleaning the optical fiber 40 after generating the operating laser pulses 110 by means of second cleaning laser pulses, wherein the laser resonator 10 of the pulsed laser system 1 is pumped by means of the pump source 20 in order to generate the second cleaning laser pulses at second cleaning energy levels between the operating energy level of 300 mJ and a second operating energy level of 1000 mJ. Specifically, as the maximum cleaning energy level MC is still 600 mJ, the second cleaning laser pulses are generated between the operating energy level of 300 mJ and 600 mJ. Afterwards, the laser resonator 10 is pumped by means of the pump source 20 after the second step of cleaning the optical fiber 40 in order to generate second operating laser pulses at the second operating energy level of 1000mJ.

**Claims**

1. Method (100) for operating a pulsed laser system, comprising the steps of:

   - pumping a laser resonator with a laser rod of the pulsed laser system by means of a pump source in order to generate operating laser pulses at an operating energy level (LO) (110), wherein the operating energy level is in a range from 100 mJ to 3000 mJ suitable to treat a target area;
   - coupling the operating laser pulses with a focusing element into an optical fiber (111), and wherein a laser beam of the operating laser pulses and cleaning laser pulses is coupled into a proximal end face of the optical fiber,
   - controlling the pump source with a control unit at an operating voltage and/or operating current such that pump energy is provided to the laser resonator at a high level for generating the operating laser pulses at an operating energy level,

   **characterized by**

   - a step of cleaning the optical fiber prior to generating the operating laser pulses by means of the cleaning laser pulses, wherein the control unit controls the pump source at one or more cleaning voltages and/or one or more cleaning currents such that pump energy is provided to the laser resonator at one or more low levels, wherein the laser resonator of the pulsed laser system is pumped by means of the pump source in order to generate the cleaning laser pulses at one or more cleaning energy levels between a laser threshold (LT) and the operating energy level (LO) (104), wherein the pump pulse duration for the cleaning laser pulses is at least 600 $\mu$s, and wherein at least part of a plurality of contaminations is removed from the proximal end face of the optical fiber.

2. Method (100) according to claim 1, wherein if the operating energy level (LO) is less than a maximum cleaning energy level (MC), then the one or more cleaning energy levels is/are in a range from the laser threshold (LT) to the operating energy level (LO) and if the operating energy level (LO) is equal to or above the maximum cleaning energy level (MC), then the one or more cleaning energy levels is/are in a range from the laser threshold (LT) to the maximum cleaning energy level (MC), wherein the maximum cleaning energy level (MC) is in a range from 400 to 1000 mJ.

3. Method (100) according to claim 2, wherein if the operating energy level (LO) is below the maximum cleaning energy level (MC), the method comprises a second step of cleaning the optical fiber after generating the first operating laser pulses (110) by means of second cleaning laser pulses, wherein the laser resonator of the pulsed laser system is pumped by means of the pump source in order to generate the second cleaning laser pulses at one or more second cleaning energy levels between the first operating energy level (LO) and a second operating energy level, wherein the second operating energy level is higher than the first operating energy level.

4. Method (100) according to any one of the preceding claims, wherein the cleaning laser pulses are generated as a sequence of laser pulse groups, wherein each one of the laser pulse groups comprises at least one of the cleaning laser pulses, and wherein the cleaning energy level is increased from one laser pulse group to a following laser pulse group (105).

5. Method (100) according to claim 4, wherein the cleaning energy level is increased by a linear factor and/or an exponential factor from the one laser pulse group to the following laser pulse group (108).

6. Method (100) according to claim 4 or 5, wherein each one of the laser pulse groups comprises at least two of the cleaning laser pulses at the same cleaning energy level (107).

7. Method (100) according to any one of the preceding claims, wherein the optical fiber is a reusable optical fiber and is used by an operator several times.

8. Method (100) according to any one of the preceding claims, wherein the method further comprises prior to cleaning the optical fiber a step of heating up a laser rod of the pulsed laser system by means of pre-pumping-pulses of the pump source, wherein the pump source is operated below the laser threshold (101).

9. Method (100) according to any one of the preceding claims, wherein the pump source is controlled by the control unit selectively at the operating voltage and/or operating current or at the one or more cleaning voltages and/or one or more cleaning currents such that the pump energy is provided to the laser resonator selectively at the high level for generating the operating laser pulses at the operating energy level or at the one or more low levels for generating the cleaning laser pulses at the one or more cleaning energy levels.

10. Pulsed laser system (1), comprising:

- a laser resonator (10) with a laser rod (11) configured to generate operating laser pulses at an operating energy level, wherein the operating energy level is in a range from 100 mJ to 3000 mJ suitable to treat a target area;
- a pump source (20) configured to provide pump energy (P) to the laser resonator (10);
- an optical fiber (40) , wherein the optical fiber (40) comprises a proximal end face (40a) for coupling a laser beam (B) of the operating laser pulses and cleaning laser pulses into the optical fiber (40);
- a focusing element (30) for coupling the operating laser pulses into the optical fiber (40); and
- a control unit (50) configured to control the pump source (20) at an operating voltage and/or operating current such that the pump energy (P) is provided to the laser resonator (11) at a high level for generating the operating laser pulses at an operating energy level,

**characterized in that**

the control unit (50) is configured to control the pump source (20) at one or more cleaning voltages and/or one or more cleaning currents such that the pump energy (P) is provided to the laser resonator (11) at one or more low levels for generating the cleaning laser pulses prior to the operating laser pulses at one or more cleaning energy levels between a laser threshold and the operating energy level for cleaning the optical fiber (40) in order to at least partly remove a plurality of contaminations from the proximal end face (40a) of the optical fiber (40), wherein the pump pulse duration for the cleaning laser pulses is at least 600 $\mu$s.

11. Pulsed laser system (1) according to claim 10, configured to perform the method (100) for operating a pulsed laser system according to any one of claims 1-9, particularly wherein a data storage device is associated with the reusable optical fiber.

**Patentansprüche**

1. Verfahren (100) zum Betreiben eines gepulsten Lasersystems, mit den Schritten:

- Pumpen eines Laserresonators mit einem Laserstab des gepulsten Lasersystems durch eine Pumpquelle zur Erzeugung von Arbeitslaserpulsen bei einem Arbeitsenergiepegel (LO) (110), wobei der Arbeitsenergiepegel in einem Bereich von 100 mJ bis 3000 mJ ist, der zur Behandlung eines Zielbereichs geeignet ist;
- Einkoppeln der Arbeitslaserpulse in eine optische Faser (111) mittels eines fokussierenden Elements, und wobei ein Laserstrahl der Arbeitslaserpulse und Reinigungslaserpulse in eine proximale Stirnfläche der optischen Faser eingekoppelt wird,
- Steuern der Pumpquelle mit einer Steuereinheit auf eine Arbeitsspannung und/oder einen Arbeitsstrom, sodass Pumpenergie dem Laserresonator bei einem hohen Pegel zur Erzeugung der Arbeitslaserpulse bei dem Arbeitsenergiepegel zugeführt wird,

**gekennzeichnet durch**

- einen Schritt zum Reinigen der optischen Faser durch Reinigungslaserpulse vor dem Erzeugen der Arbeitslaserpulse, wobei die Steuereinheit die Pumpquelle auf eine oder mehrere Arbeitsspannungen und/oder einen oder mehrere Arbeitsströme steuert, sodass Pumpenergie dem Laserresonator bei einem oder mehreren niedrigen Pegeln zugeführt wird, wobei der Laserresonator des gepulsten Lasersystems durch die Pumpquelle derart gepumpt wird, dass die Reinigungslaserpulse bei einem oder mehreren Reinigungsenergiepegeln zwischen einer Laserschwelle (LT) und dem Arbeitsenergiepegel (LO) (104) erzeugt werden, wobei die Pumppulsdauer für die Reinigungslaserpulse zumindest 600 $\mu$s ist, und wobei zumindest ein Teil einer Vielzahl von Kontaminationen von der proximalen Stirnfläche der optischen Faser entfernt wird.

2. Verfahren (100) nach Anspruch 1, wobei, wenn der Arbeitsenergiepegel (LO) kleiner als ein maximaler Reinigungsenergiepegel (MC) ist, dann der eine oder die mehreren Reinigungsenergiepegel im Bereich von der Laserschwelle(LT) bis zu dem Arbeitsenergiepegel (LO) liegen, und wenn der Arbeitsenergiepegel (LO) gleich oder über dem maximalen Reinigungsenergiepegel (MC) ist, dann der eine oder die mehreren Reinigungsenergiepegel in einem Bereich von der Laserschwelle (LT) bis zu dem maximalen Reinigungsenergiepegel (MC) liegen, wobei der maximale Reinigungsenergiepegel (MC) in einem Bereich von 400 bis 1000 mJ liegt.

3. Verfahren (100) nach Anspruch 2, wobei, wenn der Arbeitsenergiepegel (LO) unter dem maximalen Reinigungsenergiepegel (MC) liegt, das Verfahren einen zweiten Schritt der Reinigung der optischen Faser nach der Erzeugung der ersten Arbeitslaserpulse (110) mittels zweiter Reinigungslaserpulse umfasst, wobei der Laserresonator des gepulsten Lasersystems mittels der Pumpquelle derart gepumpt wird, dass die zweiten Reinigungslaserpulse mit einem oder mehreren zweiten Reinigungsenergiepegeln zwischen dem ersten Arbeitsenergiepegel (LO) und einem zweiten Arbeitsenergiepegel erzeugt werden, wobei der zweite Arbeitsenergiepegel höher ist als der erste Arbeitsenergiepegel.

4. Verfahren (100) nach einem der vorhergehenden Ansprüche, wobei die Reinigungslaserpulse als eine Sequenz aus Laserpulsgruppen erzeugt werden, wobei jede der Laserpulsgruppen mindestens einen der Reinigungslaserpulse umfasst, und wobei der Reinigungsenergiepegel von einer Laserpulsgruppe zu einer folgenden Laserpulsgruppe (105) erhöht wird.

5. Verfahren (100) nach Anspruch 4, wobei der Reinigungsenergiepegel um einen linearen Faktor und/oder einen exponentiellen Faktor von der einen Laserpulsgruppe zu der folgenden Laserpulsgruppe (108) erhöht wird.

6. Verfahren (100) nach Anspruch 4 oder 5, wobei jede der Laserpulsgruppen mindestens zwei der Reinigungslaserpulse mit dem gleichen Reinigungsenergiepegel (107) umfasst.

7. Verfahren (100) nach einem der vorhergehenden Ansprüche, wobei die optische Faser eine wiederverwendbare optische Faser ist und mehrmals durch einen Benutzer verwendet wird.

8. Verfahren (100) nach einem der vorhergehenden Ansprüche, wobei das Verfahren weiter vor dem Reinigen der optischen Faser einen Schritt zur Erwärmung eines Laserstabs des gepulsten Lasersystems mittels Vorpumppulsen der Pumpquelle umfasst, wobei die Pumpquelle unterhalb der Laserschwelle (101) betrieben wird.

9. Verfahren (100) nach einem der vorhergehenden Ansprüche, wobei die Pumpquelle durch die Steuereinheit selektiv auf die Arbeitsspannung und/oder den Arbeitsstrom oder auf die eine oder mehreren Reinigungsspannungen und/oder die einen oder mehreren Reinigungsströme derart gesteuert wird, dass die Pumpenergie dem Laserresonator selektiv bei dem hohen Pegel zur Erzeugung der Arbeitslaserpulse bei dem Arbeitsenergiepegel oder mit dem einem oder den mehreren tiefen Pegeln zur Erzeugung der Reinigungslaserpulse bei dem einen oder den mehreren Reinigungsenergiepegeln zugeführt wird.

10. Gepulstes Lasersystem (1), mit:

   - einem Laserresonator (10) mit einem Laserstab (11), der ausgebildet ist, Arbeitslaserpulse mit einem Arbeitsenergiepegel zu erzeugen, wobei der Arbeitsenergiepegel in einem Bereich von 100 mJ bis 3000 mJ ist, der zur Behandlung eines Zielbereichs geeignet ist;
   - einer Pumpquelle (20), die ausgebildet ist, dem Laserresonator (10) Pumpenergie (P) zuzuführen;
   - einer optischen Faser (40), wobei die optische Faser (40) eine proximale Stirnfläche (40a) zur Einkopplung eines Laserstrahls (B) der Arbeitslaserpulse und der Reinigungslaserpulse in die optische Faser (40) umfasst;
   - einem fokussierenden Element (30), das zur Einkopplung der Arbeitslaserpulse in die optische Faser (40) dient; und
   - einer Steuereinheit (50), die ausgebildet ist, die Pumpquelle (20) auf eine Arbeitsspannung und/oder einen Arbeitsstrom derart zu steuern, dass die Pumpenergie (P) dem Laserresonator (11) mit einem hohen Pegel zur Erzeugung der Arbeitslaserpulse bei einem Arbeitsenergiepegel zugeführt wird,

   **dadurch gekennzeichnet, dass**
   die Steuereinheit (50) ausgebildet ist, die Pumpquelle (20) bei einer oder mehreren Reinigungsspannungen und/oder einem oder mehreren Reinigungsströmen derart zu steuern, dass die Pumpenergie (P) dem Laserresonator (11) bei einem oder mehreren niedrigen Pegeln zur Erzeugung von Reinigungslaserpulsen bei einem oder mehreren Reinigungsenergiepegeln zwischen einer Laserschwelle und dem Arbeitsenergiepegel zur Reinigung der optischen Faser (40) vor den Arbeitslaserpulsen zugeführt wird, um eine Vielzahl von Kontaminationen von der proximalen Stirnfläche (40a) der optischen Faser (40) zumindest teilweise zu entfernen, wobei die Pumppulsdauer für die Reinigungslaserpulse zumindest 600 µs ist.

**11.** Gepulstes Lasersystem (1) nach Anspruch 10, das ausgebildet ist, das Verfahren (100) zum Betreiben eines gepulsten Lasersystems nach einem der Ansprüche 1-9 auszuführen, insbesondere, wobei eine Datenspeichervorrichtung mit der wiederverwendbaren optischen Faser verbunden ist.

**Revendications**

**1.** Procédé (100) de fonctionnement d'un système laser pulsé, comprenant les étapes suivantes :

- pompage d'un résonateur laser avec un barreau laser du système laser pulsé au moyen d'une source de pompage de manière à générer des impulsions laser de fonctionnement à un niveau d'énergie de fonctionnement (LO) (110), dans lequel le niveau d'énergie de fonctionnement est compris dans une plage de 100 mJ à 3000 mJ adaptée pour traiter une zone cible ;
- couplage des impulsions laser de fonctionnement à un élément de focalisation dans une fibre optique (111), et dans lequel un faisceau laser des impulsions laser de fonctionnement et des impulsions laser de nettoyage est couplé à une face d'extrémité proximale de la fibre optique,
- contrôle de la source de pompage avec une unité de contrôle d'une tension de fonctionnement et/ou d'un courant de fonctionnement de telle sorte que l'énergie de pompage est pourvue au résonateur laser à un niveau haut pour générer les impulsions laser de fonctionnement à un niveau d'énergie de fonctionnement,

**caractérisé par**

- une étape de nettoyage de la fibre optique avant la génération des impulsions laser de fonctionnement au moyen des impulsions laser de nettoyage, dans lequel l'unité de contrôle contrôle la source de pompage à une ou plusieurs tensions de nettoyage et/ou un ou plusieurs courants de nettoyage de telle sorte que l'énergie de pompage est fournie au résonateur laser à un ou plusieurs niveaux bas, dans lequel le résonateur laser du système laser pulsé est pompé au moyen de la source de pompage de manière à générer les impulsions laser de nettoyage à un ou plusieurs niveaux d'énergie de nettoyage entre un seuil laser (LT) et le niveau d'énergie de fonctionnement (LO) (104), dans lequel la durée d'impulsion de pompage pour les impulsions laser de nettoyage est d'au moins 600 µs, et dans lequel au moins une partie d'une pluralité de contaminations est éliminée de la face d'extrémité proximale de la fibre optique.

**2.** Procédé (100) selon la revendication 1 dans lequel, si le niveau d'énergie de fonctionnement (LO) est inférieur à un niveau d'énergie de nettoyage maximum (MC), alors lesdits un ou plusieurs niveaux d'énergie de nettoyage sont compris dans une plage allant du seuil laser (LT) au niveau d'énergie de fonctionnement (LO), et si le niveau d'énergie de fonctionnement (LO) est supérieur ou égal au niveau d'énergie de nettoyage maximum (MC), alors lesdits un ou plusieurs niveaux d'énergie de nettoyage sont compris dans une plage allant du seuil laser (LT) au niveau d'énergie de nettoyage maximum (MC), dans lequel le niveau d'énergie de nettoyage maximum (MC) est compris dans une plage de 400 à 1000 mJ.

**3.** Procédé (100) selon la revendication 2 dans lequel, si le niveau d'énergie de fonctionnement (LO) est inférieur au niveau d'énergie de nettoyage maximum (MC), le procédé comprend une deuxième étape de nettoyage de la fibre optique après la génération des premières impulsions laser de fonctionnement (110) au moyen de deuxièmes impulsions laser de nettoyage, dans lequel le résonateur laser du système laser pulsé est pompé au moyen de la source de pompage de manière à générer les deuxièmes impulsions laser de nettoyage à un ou plusieurs deuxièmes niveaux d'énergie de nettoyage entre le premier niveau d'énergie de fonctionnement (LO) et un deuxième niveau d'énergie de fonctionnement, dans lequel le deuxième niveau d'énergie de fonctionnement est supérieur au premier niveau d'énergie de fonctionnement.

**4.** Procédé (100) selon l'une quelconque des revendications précédentes, dans lequel les impulsions laser de nettoyage sont générées sous forme d'une séquence de groupes d'impulsions laser, dans lequel chacun des groupes d'impulsions laser comprend au moins l'une des impulsions laser de nettoyage, et dans lequel le niveau d'énergie de nettoyage est augmenté d'un groupe d'impulsions laser à un groupe d'impulsions laser suivant (105) .

**5.** Procédé (100) selon la revendication 4, dans lequel le niveau d'énergie de nettoyage est augmenté selon un facteur linéaire et/ou un facteur exponentiel dudit un groupe d'impulsions laser au groupe d'impulsions laser suivant (108) .

6. Procédé (100) selon la revendication 4 ou 5, dans lequel chacun desdits groupes d'impulsions laser comprend au moins deux impulsions laser de nettoyage au même niveau d'énergie de nettoyage (107).

7. Procédé (100) selon l'une quelconque des revendications précédentes, dans lequel la fibre optique est une fibre optique réutilisable et est utilisée plusieurs fois par un opérateur.

8. Procédé (100) selon l'une quelconque des revendications précédentes, dans lequel le procédé comprend en outre, avant le nettoyage de la fibre optique, une étape de chauffe d'un barreau laser du système laser pulsé au moyen d'impulsions de pré-pompage de la source de pompage, dans lequel la source de pompage est utilisée sous le seuil laser (101).

9. Procédé (100) selon l'une quelconque des revendications précédentes, dans lequel la source de pompage est contrôlée sélectivement par l'unité de contrôle à ladite tension de fonctionnement et/ou audit courant de fonctionnement ou auxdites une ou plusieurs tensions de nettoyage et/ou auxdits un ou plusieurs courants de nettoyage de telle sorte que l'énergie de pompage est fournie sélectivement au résonateur laser au niveau haut pour générer les impulsions laser de fonctionnement au niveau d'énergie de fonctionnement ou auxdits un ou plusieurs niveaux bas pour générer les impulsions laser de nettoyage auxdits un ou plusieurs niveaux d'énergie de nettoyage.

10. Système laser pulsé (1), comprenant :

- un résonateur laser (10) avec un barreau laser (11) configuré pour générer des impulsions laser de fonctionnement à un niveau d'énergie de fonctionnement, dans lequel le niveau d'énergie de fonctionnement est compris dans une plage de 100 mJ à 3000 mJ adaptée pour traiter une zone cible ;
- une source de pompage (20) configurée pour procurer de l'énergie de pompage (P) au résonateur laser (10) ;
- une fibre optique (40), dans lequel la fibre optique (40) comprend une face d'extrémité proximale (40a) pour coupler un faisceau laser (B) des impulsions laser de fonctionnement et des impulsions laser de nettoyage dans la fibre optique (40) ;
- un élément de focalisation (30) pour coupler les impulsions laser de fonctionnement dans la fibre optique (40) ; et
- une unité de contrôle (50) configurée pour contrôler la source de pompage (20) à une tension de fonctionnement et/ou un courant de fonctionnement de telle sorte que l'énergie de pompage (P) est pourvue au résonateur laser (11) à un niveau haut pour générer les impulsions laser de fonctionnement à un niveau d'énergie de fonctionnement,

**caractérisé en ce que**
l'unité de contrôle (50) est configurée pour contrôler la source de pompage (20) à une ou plusieurs tensions de nettoyage et/ou un ou plusieurs courants de nettoyage de telle sorte que l'énergie de pompage (P) est pourvue au résonateur laser (11) à un ou plusieurs niveaux bas pour générer les impulsions laser de nettoyage avant les impulsions laser de fonctionnement à un ou plusieurs niveaux d'énergie de nettoyage entre un seuil laser et le niveau d'énergie de fonctionnement pour nettoyer la fibre optique (40) de manière à éliminer au moins partiellement une pluralité de contaminations de la face d'extrémité proximale (40a) de la fibre optique (40), dans lequel la durée d'impulsion de pompage pour les impulsions laser de nettoyage est d'au moins 600 ps.

11. Système laser pulsé (1) selon la revendication 10, configuré pour mettre en œuvre le procédé (100) de fonctionnement d'un système laser pulsé selon l'une quelconque des revendications 1 à 9, en particulier dans lequel un dispositif de stockage de données est associé à la fibre optique réutilisable.

FIG. 1

FIG. 3

FIG. 2

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 9122009 B1
- CN 101867142 A
- US 20080262577 A1
- US 5269778 A